(19) Europäisches Patentamt
European Patent Office
Office européen des brevets

(11) Publication number: 0 055 248 A1

# (12) EUROPEAN PATENT APPLICATION

(21) Application number: 82200204.4

(22) Date of filing: 17.06.80

(51) Int. Cl.[3]: C 07 D 513/04, C 07 D 277/74 // C07D513/14 ,(C07D513/04, 277/ 00, 221/00),(C07D513/04, 277/00, 277/00, 221/00)

(30) Priority: 21.06.79 US 50847
31.03.80 US 130483

(43) Date of publication of application: 30.06.82 Bulletin 82/26

(84) Designated Contracting States: AT BE CH DE FR IT LI LU NL SE

(60) Publication number of the earlier application in accordance with Art. 76 EPC: 0021773

(71) Applicant: AMERICAN HOME PRODUCTS CORPORATION, 685, Third Avenue, New York, New York 10017 (US)

(72) Inventor: Gregory, Francis Joseph, 11, Cypress Lane, Berwyn Pennsylvania 19312 (US)
Inventor: Wei, Peter Hsing-Lien, 430, Ridge Lane, Springfield Pennsylvania 19064 (US)

(74) Representative: Porter, Graham Ronald et al, C/O John Wyeth & Brother Limited Huntercombe Lane South Taplow, Maidenhead Berkshire, SL6 0PH (GB)

## (54) Acetic acid derivatives.

(57) Novel compounds having the formula

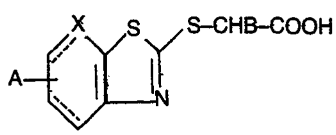

(I)

wherein A is hydrogen or a defined substituent, B is phenyl or substituted phenyl, X is CH or N and the dotted lines represent optional double bonds in the 5, 6 and 7, 8 positions subject to the proviso that α-phenyl-2-benzothiazolylthioglycolic acid is excluded are useful as chemical intermediates for the preparation of 2-phenylthiazolo-[2,3-b]benzo(and azobenzo)thiazole derivatives which are useful as immunomodulating agents.

EP 0 055 248 A1



COMPLETE DOCUMENT

## ACETIC ACID DERIVATIVES

The invention relates to novel α-phenyl-2-(aza) benzothiazolylthioglycolic acids and a process for their preparation. The compounds are useful as chemical intermediates for the preparation of 2-phenylthiazolo-[2,3-b]benzo(and azobenzo)thiazole derivatives useful as pharmaceuticals, particularly for use as modulators of the immune response.

α-Phenyl-2-benzothiazolylthioglycolic acid is disclosed in J. Org. Chem., 43, 2697-2700 (1978). The compound was prepared by reacting 2-mercaptobenzothiazole with α-bromophenylacetic acid in the presence of triethylamine. The product was cyclized with acetic anhydride in the presence of triethylamine according to the equation:

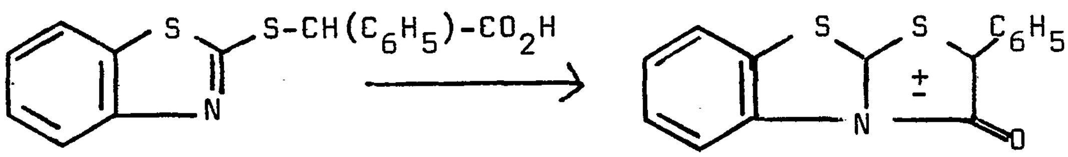

(A) (B)

The cyclization product (B) was subjected to various reactions whereby several new compounds were made. However, no use for these new compounds was given. Thus the reference envisages no technical use for α-phenyl-2-benzothiazolylthioglycolic acid (A) and its cyclization product (B).

The invention is based upon the finding that novel derivatives of α-phenyl-2-benzothiazolylthioglycolic acid are useful as chemical intermediates for the preparation of novel 2-phenylthiazolo[2,3-b]benzo (and azobenzo) thiazole derivatives useful as agents for modulating the immune response in warm blooded animals.

The invention provides novel compounds having the formula

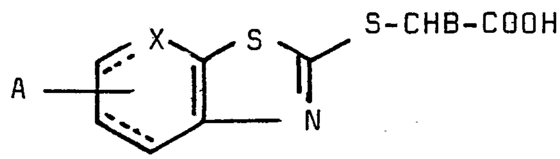

(I)

wherein A is hydrogen, halo, amino, amino protected with a removable protecting group, lower alkyl, lower alkoxy, trifluoromethyl, hydroxy or hydroxy protected by a removable protecting group; B is phenyl or substituted phenyl, for instance, phenyl substituted by one or more substituents selected from fluorine, chlorine, bromine, lower alkyl, lower alkoxy, amino, amino protected by a removable protecting group, nitro and trifluoromethyl; X is CH or N and the dotted lines represent optional double bonds in the 5,6 and 7, 8 positions subject to the proviso that when the group having the formula

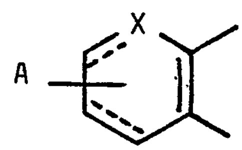

(II)

is ortho-phenylene then B is other than phenyl.

The 2-phenylthiazolo[2,3-b]benzo(and azobenzo) thiazole derivatives obtainable as end compounds from the compounds of the invention have the formula III or IV:

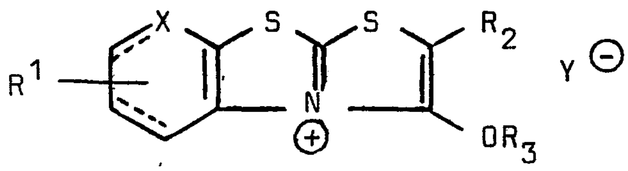

(III)

or

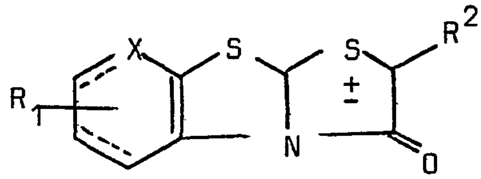

(IV)

wherein $R_1$ is hydrogen, halo, amino, lower alkyl, lower alkoxy, trifluoromethyl or hydroxy; $R_2$ is phenyl or substituted phenyl, for instance, phenyl substituted by one or more substituents selected from fluorine, chlorine, bromine, lower alkyl, lower alkoxy, amino, nitro and trifluoromethyl; $R_3$ is hydrogen or lower alkanoyl; X is CH or N; Y is a pharmaceutically acceptable anion, for instance, trifluoroacetate or a halide; the dotted lines represent optional double bonds in the 5,6 and 7,8 positions; subject to the proviso that where the group having the formula

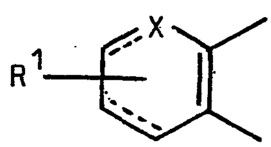

(IIa)

is ortho-phenylene, then $R_2$ is other than phenyl.

A first subgenus of these compounds consists in those having formula III or IV, wherein $R_2$ is phenyl or phenyl substituted with a substituent selected from fluorine, chlorine, bromine, lower alkyl, lower alkoxy, amino, nitro and trifluoromethyl; and Y is $CF_3CO_2^{\ominus}$ or halide.

A second subgenus of these compounds consists in those having formula III or IV, wherein $R_3$ is hydrogen and Y is $CF_3CO_2^{\ominus}$ or halide.

$R_1$ is preferably hydrogen, halo or lower alkoxy, $R_2$ is preferably phenyl or halophenyl , $R_3$ is preferably hydrogen or acetyl. Y is preferably $CF_3CO_2^{\ominus}$ or halide.

The term "lower alkyl" when used herein includes straight and branched chain hydrocarbon radicals having from 1 to 6 carbon atoms. The terms "lower alkoxy" and "lower alkanoyl" in like manner designate radicals in which the hydrocarbon portion has 1 to about 6 carbon atoms.

The terms "halo" and "halide" when used herein refer to radicals of the elements fluorine, chlorine and bromine and chlorine and bromine respectively.

The compounds having formula I can be prepared in known manner. In particular a compound having the formula V

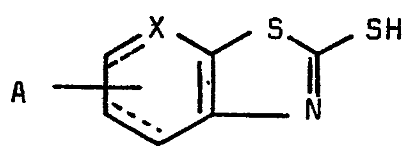

(V)

(wherein A, X and the dotted lines are as defined above) is reacted with a compound having the formula Hal-CHB-COOH (VI) (in which Hal is chlorine or bromine and B is as defined above). If the reaction is carried out in the presence of a condensing agent, for instance, acetic anhydride or trifluoroacetic anhydride, a 2-phenylthiazolo[2,3-*b*]benzo (or azobenzo)thiazole derivative may be formed. Accordingly the use of a condensing agent should be avoided for the preparation of the compounds having formula I.

The 2-phenylthiazolo[2,3-*b*]benzo(and azobenzo) thiazole derivatives may be prepared by a process comprising

(a) cyclizing a compound having the formula

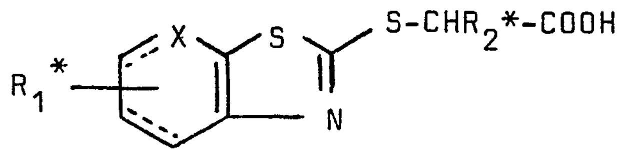

(VI)

(wherein X and the dotted lines are as defined above and $R_1$* and $R_2$* are the same as $R_1$ and $R_2$ respectively save that, where necessary or desired, an amino or hydroxy group may be protected with a removable protecting group) and, if desired,

(b) a compound having the formula

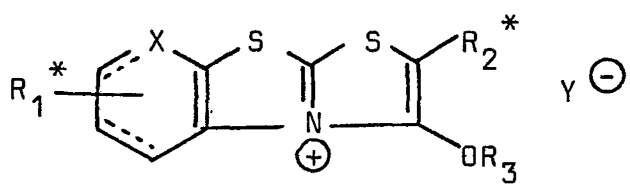

(VII)

(wherein $R_1$*, $R_2$*, $R_3$, X, and the dotted lines are as defined above and Y is an anion) is subjected to (i) deacylation and removal of the acid HY, if $R_3$ is lower alkanoyl, or (ii) removal of the acid HY, if $R_3$ is hydrogen; or

(c) a compound having the formula

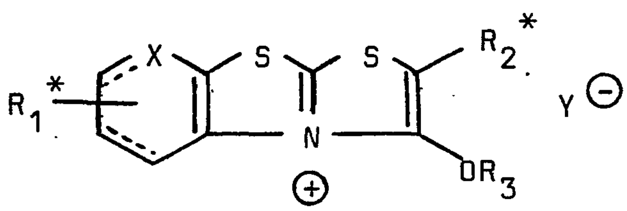

(VII)

(wherein $R_1$*, $R_2$*, X and the dotted lines are as defined above, Y is a pharmaceutically acceptable anion and $R_3$ is lower alkanoyl) is subjected to deacylation to form a compound where $R_3$ is hydrogen; and, where necessary, a removable protecting group is removed.

The product obtained by the cyclization steps depends upon the reactants used and the reaction conditions. Where the reactants used do not serve to provide a suitable anion Y or where the cyclization is carried out with a base such as triethylamine the cyclization product is in the form of a mesoionic didehydro derivative. Where a salt of formula III is prepared, the anion Y is preferably provided by one of the reactants used. For example, use of trifluoroacetic anhydride as condensing agent may give a trifluoroacetate salt. Where the cyclization product is in the form of a salt, it may exist in the form of a 3-hydroxy compound, or in the form in which $R_3$ is in the residue of the condensing agent.

In those instances where $R_1$ is amino or hydroxy, or $R_2$ contains amino or hydroxy, the groups can be protected from acylation during the cyclization reaction by use of conventional protecting groups, for example, tosyl, benzyloxycarbonyl, tert-butyloxycarbonyl and the like for the amino group and acetyl, benzoyl, tert-butyl, benzyl and the like for the hydroxy group. The protecting groups can be readily removed by conventional techniques after cyclization is complete.

The cyclization step uses a condensing agent. As condensing agents there may be used acetic anhydride, which may be in admixture with acetic acid, or trifluoroacetic anhydride and the like. The cyclization may be carried out by heating the reactants in an organic solvent such as, for example, acetone.

Where a salt is formed where $R_3$ is lower alkanoyl residue from the condensing agent and it is desired to obtain the product where $R_3$ is hydrogen in accorance with step (c), the condensing agent residue can readily be removed by conventional means, as for example deacylation by heating in certain solvents.

The transformation of step (b) can be carried out by dissolving the salt in a methylene chloride/water mixture, separating the organic and aqueous layers and concentrating the organic (methylene chloride) layer to recover the mesoionic didehydro derivative, which can then be further purified by recrystallization.

Although it is recommended to avoid undesired acylation by protecting an amino or hydroxy group for the cyclization steps, the presence of a removable protecting group in the compounds of formula VII for steps (b) and (c) is optional.

The preparation and use of the compounds of the invention is preferably carried out as follows. A mercapto compound (VIII) is reacted with a suitably substituted α-haloacetic acid (IX) in the absence of a condensing agent to prepare a suitably substituted 2-benzothiazolylthioglycolic acid (X) and cyclization of the product (X) is carried out in a subsequent step:

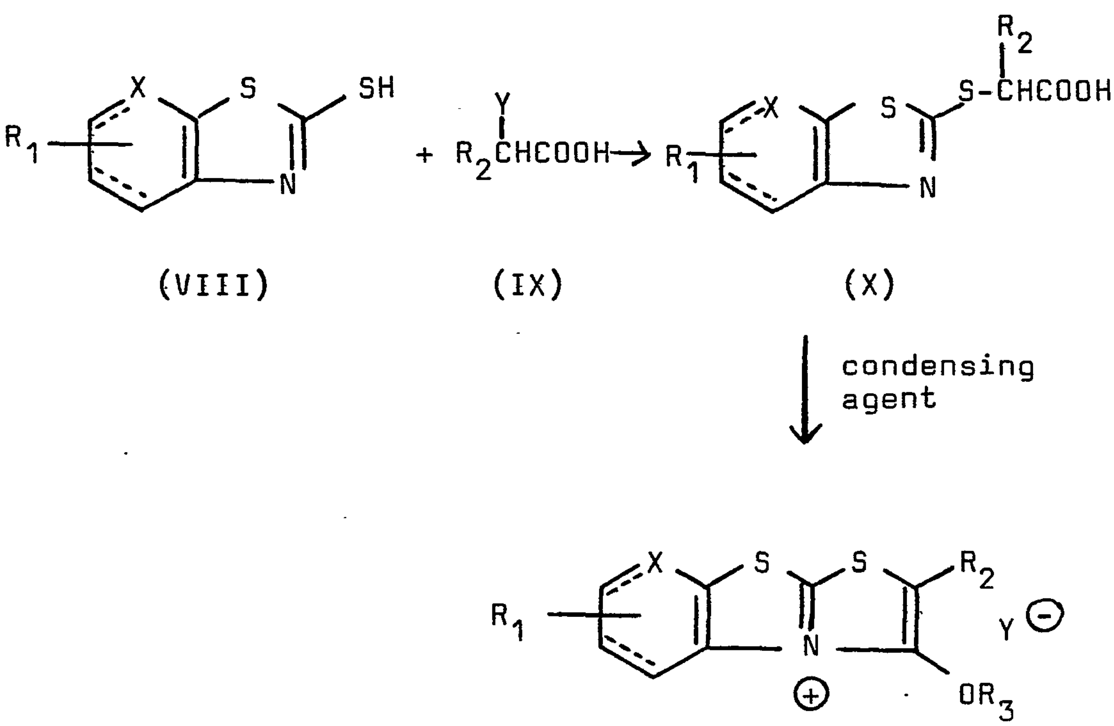


(VIII) (IX) (X)

(III)

In those instances where $R_1$ is amino or hydroxy, the latter groups can be protected from acylation during the cyclization reaction by use of conventional protecting groups.

The compounds of formulae III and IV are immunomodulators of high selectivity, having particular activity on the cell-mediated immune system without impeding the humoral immune mechanisms. The compounds of formulae III and IV have therapeutic application in a variety of situations in which immunomodulation is indicated. Thus, the compounds of formulae III and IV permit the host to accept the graft without destroying the host's immunity to other infections. The compounds of formulae III and IV are also useful in the treatment of autoimmune diseases, such as systemic lupus erythematosus (SLE). Further, the compounds of formulae III and IV inhibit the production of the immunoglobulins, which are so pathologic to autoimmune disease such as SLE, as well as the production of antigen-antibody complexes which are the causative agents of renal and inflammatory processes in arthritis and auto-iiune diseases. Thus, the compounds of formulae III and IV are also useful in the treatment of such conditions as rheumatoid arthritis.

The thiazolo[2,3-b]benzo(and azobenzo)thiazole derivatives having formulae III and IV are described and claimed in our copending Application No. 80302034.6. Further particulars such as the pharmacological evaluation of such compounds, dosages and pharmaceutical compositions are also described in the aforesaid application.

The following Example illustrates the invention:

# EXAMPLE

## 2-Phenylthiazolo[2',3'-2,3]thiazolo[5,4-b]-pyridin-3(2H)-one mesoionic didehydro derivative

A. α-(Thiazolo [5,4-b]-pyridin-2-yl-thio)phenyl acetic acid

6.5g (0.03 moles) α-bromophenylacetic acid and 6.2 g. (0.03 moles) potassium salt of 2-mercaptothiazolo[5,4-b]-pyridine are suspended in 200 ml. acetone and the mixture is stirred at room temperature overnight. The mixture is filtered and the filtrate concentrated to dryness. The resulting compound is an oil and is not isolated.

B. 3-Hydroxy-2-phenylthiazolo[2',3'-2,3]thiazolo-[5,4-b]pyridinium trifluoroacetate

The compound of A above is dissolved in ether and 20 ml. of trifluoroacetic anhydride is added to the solution. The solid which forms immediately is collected, washed with ether and dried. The product weighs 9.7 g.

C. 2-Phenylthiazolo[2',3'-2,3]thiazolo[5,4-b]pyridin-3(2H)-one mesoionic didehydro derivative

8.5 g. of the compound of B above is suspended in a mixture of 200 ml. methylene chloride and 150 ml. water and the mixture is stirred at room temperature for 30 minutes. The orange solid is collected and dried at $100^{o}$ *in vacuo*. The compound weighing 5.0 g (80% yield) at $216-218^{o}C$. (dec.)

| | | | | |
|---|---|---|---|---|
| Analysis for: | $C_{14}H_8N_2OS_2$ | | | |
| Calculated: | C, 59.13; | H, 2.83; | N, 9.85; | S, 22.55 |
| Found: | C, 59.06; | H, 2.90; | N, 9.79; | S, 22.14 |

CLAIMS

1. A compound having the formula

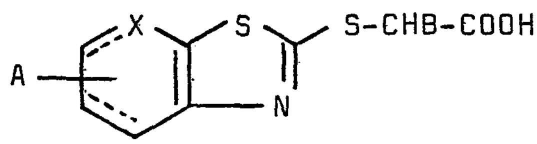

(I)

wherein A is hydrogen, halo, amino, amino protected by a removable protecting group, lower alkyl, lower alkoxy, trifluoromethyl, hydroxy or hydroxy protected by a removable protecting group; B is phenyl or substituted phenyl; X is CH or N and the dotted lines represent optional double bonds in the 5,6 and 7,8 positions subject to the proviso that when the group having the formula

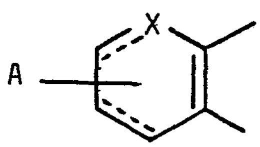

(II)

is ortho-phenylene then B is other than phenyl.

2. A compound having the formula

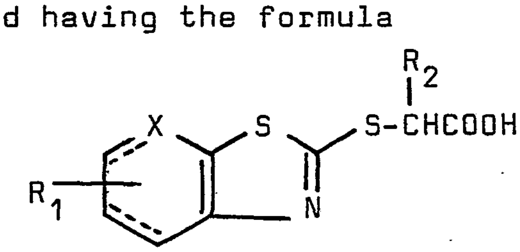

(X)

wherein $R_1$ is hydrogen, halo, amino, lower alkyl, lower alkoxy, trifluoromethyl or hydroxy and $R_2$ is phenyl or phenyl substituted with a substituent selected from fluorine, chlorine, bromine, lower alkyl, lower alkoxy, amino, nitro and trifluoromethyl and X and the dotted lines are as defined in Claim 1 subject to the proviso that where the group having the formula IIa

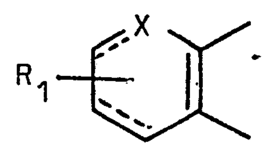

(IIa)

is ortho-phenylene, then $R_2$ is other than phenyl.

3. A compound as claimed in Claim 2, wherein $R_1$ is hydrogen, halo or lower alkoxy.

4. A compound as claimed in Claim 2 or 3, wherein $R_2$ is phenyl or halophenyl.

5. α-(Thiazolo[5,4-b]pyridin-2-ylthio)phenylacetic acid.

6. A process for the preparation of a compound as claimed in Claim 1, wherein a compound having the formula

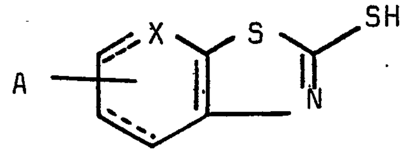

(V)

(wherein A, X and the dotted lines are as defined in Claim 1) is reacted with a compound having the formula

$$\text{Hal-CHB-COOH} \qquad (VI)$$

(wherein Hal is chlorine or bromine and B is as defined in Claim 1).

7. A process for the preparation of α-(thiazolo [5,4-b]pyridin-2-ylthio)phenylacetic acid, wherein α-bromophenylacetic acid is reacted with the potassium salt of 2-mercaptothiazolo[5,4-b] pyridine.

CLAIMS FOR AUSTRIA

1. A process for the preparation of a compound having the formula

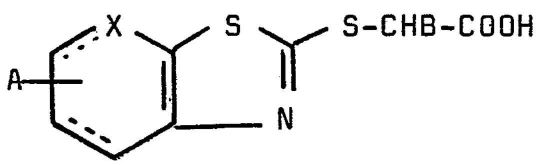

(I)

wherein A is hydrogen, halo, amino, amino protected by a removable protecting group, lower alkyl, lower alkoxy, trifluoromethyl, hydroxy or hydroxy protected by a removable protecting group; B is phenyl or substituted phenyl; X is CH or N and the dotted lines represent optional double bonds in the 5,6 and 7,8 positions subject to the proviso that when the group having the formula

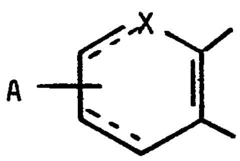

(II)

is ortho-phenylene then B is other than phenyl, characterised in that a compound having the formula

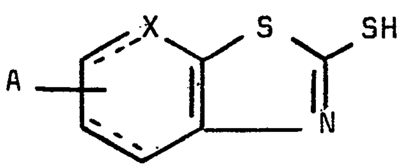

(V)

(wherein A, X and the dotted lines are as defined above) is reacted with a compound having the formula

Hal-CHB-COOH (VI)

(wherein Hal is chlorine or bromine and B is as defined above).

2. A process as claimed in Claim 1, characterised in that A is hydrogen, halo, amino, lower alkyl, lower alkoxy, trifluoromethyl or hydroxy and B is phenyl or phenyl substituted with a substituent selected from fluorine, chlorine, bromine, lower alkyl, lower alkoxy, amino, nitro and trifluoromethyl.

3. A process as claimed in Claim 2, wherein $A_1$ is hydrogen, halo or lower alkoxy.

4. A process as claimed in Claim 2 or 3, wherein $B_2$ is phenyl or halophenyl.

5. A process for the preparation of α-(thiazolo[5,4-b]-2-ylthio)phenylacetic acid, characterised in that α-bromophenylacetic acid is reacted with the potassium salt of 2-mercaptothiazolo[5,4-b]pyridine.

0055248

European Patent Office

# EUROPEAN SEARCH REPORT

Application number

EP 82 20 0204

## DOCUMENTS CONSIDERED TO BE RELEVANT

| Category | Citation of document with indication, where appropriate, of relevant passages | Relevant to claim |
|---|---|---|
| A | GB - A - 1 111 492 (AGFA)<br>* Page 1, lines 45-52; page 2, lines 1-6 *<br>-- | 1 |
| EX | EP - A - 0 030 632 (MERCK)<br>* Examples 51 and 64 *<br>---- | 1 |

CLASSIFICATION OF THE APPLICATION (Int. Cl. 3)

C 07 D 513/04
277/74
513/14
(C 07 D 513/04
277/00
221/00)
(C 07 D 513/04
277/00
277/00
221/00)

TECHNICAL FIELDS SEARCHED (Int.Cl. 3)

C 07 D 513/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 06-04-1982 | ALFARO |

EPO Form 1503.1 06.78